# EUROPEAN PATENT SPECIFICATION

(11) **EP 2 413 920 B1**
(45) Date of publication and mention of the grant of the patent: **21.08.2013**
(21) Application number: 10715153.2
(22) Date of filing: 02.04.2010
(51) Int. Cl.: A61P 35/00, A61P 35/04, A61K 36/9066, A61K 31/337

(54) **Curcuminoids in combination with Docetaxel for use in the treatment of cancer and tumour metastasis**
Kurkuminoide in kombination mit Docetaxel zur Behandlung von Krebs und Tumormetastasen
Curcuminoïdes en combinaison avec Docétaxel pour le traitement d'un cancer et d'une métastase tumorale

(30) Priority: 03.04.2009 EP 09305282
(43) Date of publication of application: 08.02.2012
(73) Proprietor: INSERM (Institut National de la Santé et de la Recherche Médicale), 75013 Paris (FR)
(72) Inventor: BARTHOMEUF, Chantal, F-63001 Cedex1 Clermont Ferrand (FR); CHOLLET, Philippe, F-63011 Cedex 1 Clermont Ferrand (FR); BAYET-ROBERT, Mathilde, F-63011 Cedex1 Clermont Ferrand (FR)
(74) Representative: Hirsch, Denise Marie
(86) International application number: PCT/EP2010/054451
(87) International publication number: WO 2010/115852

(56) References cited:
- LIMTRAKUL ET AL.: BMC CANCER, [Online] vol. 4, no. 13, 17 April 2004 (2004-04-17) , XP002590812 Retrieved from the Internet: URL:http://www.biomedcentral.com/1471-2407 /4/13> [retrieved on 2010-01-07]
- UM Y ET AL: "Synthesis of curcumin mimics with multidrug resistance reversal activities" BIOORGANIC & MEDICINAL CHEMISTRY, PERGAMON, GB LNKD- DOI:10.1016/J.BMC.2008.02.012, vol. 16, no. 7, 1 April 2008 (2008-04-01), pages 3608-3615, XP022593468 ISSN: 0968-0896 [retrieved on 2008-02-08]
- DATABASE MEDLINE [Online] US NATIONAL LIBRARY OF MEDICINE (NLM), BETHESDA, MD, US; September 2001 (2001-09), ZHANG H ET AL: "[Study on active constituents of traditional Chinese medicine reversing multidrug resistance of tumor cells in vitro]" XP002590813 Database accession no. NLM11799777 & ZHONG YAO CAI = ZHONGYAOCAI = JOURNAL OF CHINESE MEDICINAL MATERIALS SEP 2001 LNKD- PUBMED:11799777, vol. 24, no. 9, September 2001 (2001-09), pages 655-657, ISSN: 1001-4454
- PATEL BHAUMIK B ET AL: "Curcumin enhances the effects of 5-fluorouracil and oxaliplatin in mediating growth inhibition of colon cancer cells by modulating EGFR and IGF-1R." INTERNATIONAL JOURNAL OF CANCER. JOURNAL INTERNATIONAL DU CANCER 15 JAN 2008 LNKD- PUBMED:17918158, vol. 122, no. 2, 15 January 2008 (2008-01-15), pages 267-273, XP002590814 ISSN: 1097-0215
- HERBST ROY S ET AL: "Mode of action of docetaxel - a basis for combination with novel anticancer agents." CANCER TREATMENT REVIEWS OCT 2003 LNKD- PUBMED:12972359, vol. 29, no. 5, October 2003 (2003-10), pages 407-415, XP002590815 ISSN: 0305-7372
- BAE MOON-KYOUNG ET AL: "Curcumin inhibits hypoxia-induced angiogenesis via down-regulation of HIF-1." ONCOLOGY REPORTS JUN 2006 LNKD- PUBMED:16685395, vol. 15, no. 6, June 2006 (2006-06), pages 1557-1562, XP002590816 ISSN: 1021-335X
- PURKAYASTHA S ET AL: "Curcumin blocks brain tumor formation" BRAIN RESEARCH, ELSEVIER, AMSTERDAM, NL LNKD- DOI:10.1016/J.BRAINRES.2009.01.066, vol. 1266, 11 February 2009 (2009-02-11), pages 130-138, XP026081452 ISSN: 0006-8993 [retrieved on 2009-02-11]
- KEMPER E M ET AL: "Improved penetration of docetaxel into the brain by co-administration of inhibitors of P-glycoprotein." EUROPEAN JOURNAL OF CANCER (OXFORD, ENGLAND : 1990) MAY 2004 LNKD- PUBMED:15110893, vol. 40, no. 8, May 2004 (2004-05), pages 1269-1274, XP002590817 ISSN: 0959-8049
- SIMONI ET AL: "Antitumor effects of curcumin and structurally beta-diketone modified analogs on multidrug resistant cancer cells" BIOORGANIC & MEDICINAL CHEMISTRY LETTERS, PERGAMON, ELSEVIER SCIENCE, GB LNKD- DOI:10.1016/J.BMCL.2007.11.021, vol. 18, no. 2, 13 November 2007 (2007-11-13), pages 845-849, XP022424762 ISSN: 0960-894X
- LIMTRAKUL PORNNGARM: "Curcumin as chemosensitizer." 2007, ADVANCES IN EXPERIMENTAL MEDICINE AND BIOLOGY 2007 LNKD- PUBMED:17569216, VOL. 595, PAGE(S) 269 - 300 , XP009135834 ISSN: 0065-2598 the whole document

## Description

The present invention is described in claims 1-9 and relates to the field of cancer and tumour metastasis treatment.

### BACKGROUND OF THE INVENTION:

Cancer is a deadly disease causing significant morbidity and mortality. In 2007, breast cancer has been estimated as the first (worldwide, developed countries) or second (developing countries) cause of cancer-related death in female (Garcia M et al. Global cancer Facts and Figures 2007, Edited by AACR.) It affects approximately one out of thirty-nine to one out of three women who reach age ninety in the Western world. Prostate cancer has been estimated to be the first cause of new cancer cases and the fifth cause of cancer-related death in males from developed countries (Garcia M et al. 2007). Finally, the most malignant tumour of the human nervous system: glioblastoma [World Health Organization (WHO) Grade IV] is one of the most lethal tumours. It is the most common cause of cancer-related death in childwood and the first cause of primary brain tumour in adult. The majority of glioblastoma patients are treated with surgery, radiation or by alkylating agents-based chemotherapy. However, glioblastoma patients still have very poor prognosis due to incomplete resection and resistance to radio- and chemotherapy largely due to endothelial cells of the « blood brain barrier » (BBB) that limits entry and effectiveness of chemotherapeutics through expression of P-glycoprotein (see Barthomeuf et al. 2008 for review). Novel therapeutic approaches are therefore needed to prolong patient survival.

Docetaxel (Taxotere®, DTX) has demonstrated a broad spectrum of antitumour activities and has proved clinical efficacy in multiple tumour types, including advanced (metastatic) ovarian, breast and prostate cancers. It is already the reference treatment of advanced breast (all lines) or prostate (first line) cancers (see Montero A et al. Lancet Oncol 2005, 6 : 229-39 for review on clinical data). Singly, the clinical value of this microtubule-stabilizing agent is restricted by PTEN inactivation (Saunders J. et al. Proc Amer Assoc Cancer Res, 2004, 45 abstract 5610), by EGFR-induced up-regulation of survivin gene (Pen et al. 2006), by cumulative systemic toxicity after prolonged high dose-therapy and by VEGFR up-regulation and Bcl-2 or Pgp overexpression (Hernandez-Vargas H. et al Oncogene 2007, 26: 2902-13). Pgp -mediated DTX efflux is mainly responsible for the limited efficacy of DTX on colon tumours and on brain tumours protected by endothelial cells of the BBB (Investigator brochure, 2002). The clinical efficacy of DXT is enhanced by combination with (i) inhibitors of EGFR signaling such as trastuzumab (Herceptin®, a monoclonal antibody directed against HER-2), or cetuximab (Erbitux®, a monoclonal antibody directed against HER-1), (ii) by farnesyl transferase inhibitors, (iii) by the 5-fluorouracile (5-FU) prodrug: capecitabine (Xeloda®) or (iv) by the Bcl-2 siRNA: oblimersen sodium (Genasense®) (see Herbst RS and Khuri FR, Cancer Treat Rev 2003, 29: 407-15 for review).

Curcumin (diferuloylmethane) is the major component of the yellow spice turmeric mainly extracted from the rhizomes of Curcuma longa and traditionally used as a spice in India. Curcumin has demonstrated cancer chemopreventive activity in several clinical trials (see Aggarwald BB et al. Anticancer Res. 2003, 23: 363-98 for review) including in patients with high-risk or pre-malignant lesions. No toxicity has been observed at dose up to 8,000 mg/d in these patients (Cheng AL et al. Anticancer Res 2001, 4B : 2895-900) or in patients with advanced colorectal cancer resistant to conventional therapy treated by curcumin at doses ranging between 36-180 mg/day. Interestingly, a stabilisation has been observed in 5/15 patients with colorectal cancer (Sharma RA et al. 2001, 7 : 1894-900). Curcumin and several natural curcuminoids has proved capacity to dose-dependently inhibit Pgp transport function and expression (Anuchapreeda S et al. Biochem Pharmacol. 2002, 64:573-82 ; Limtrakul P et al. BMC Cancer. 2004 April 17, 4-13 ; Limtrakul P. Adv Exp Med Biol. 2007; 595: 269-300). Synthetic curcumin-derivatives reversing multidrug resistance have also been produced (Um Y et al. Bioorg Med Chem. 2008 16(7):3608-15). The reversal effects of curcumin have been attributed to lower PI3K/Akt/NFkB signaling (Choi BH et al Cancer Lett. 2008, 259:111-8.).

However up to now, DTX and curcuminoids have never been associated clinically for treatment of cancers.

### SUMMARY OF THE INVENTION:

The present invention relates to the use of curcuminoids for enhancing the clinical efficacy of Docetaxel (Taxotere®, DTX) for use in the treatment of cancers and metastases.

### DETAILED DESCRIPTION OF THE INVENTION:

The present invention relates to the use of curcuminoids for enhancing the clinical efficacy of Docetaxel (Taxotere®, DTX) for use in the treatment of cancers and metastases.

As used herein the term "Docetaxel" or "DTX" refers to (2R,3S)-N-carboxy-3-phenylisoserine, N-tert-butyl ester, 13-ester with 5, 20-epoxy-1,2, 4, 7, 10, 13-hexahydroxytax-11-en-9-one 4-acetate 2-benzoate, trihydrate. (CAS number: 114977-28-5)

The term "curcuminoid" encompasses natural curcumin (diferuloylmethane, feruloyl (1E,6E)-1,7-bis (4-hydroxy-3-methoxyphenyl)-1,6-heptadiene-3,5-dione) and structurally-derived compounds. Curcuminoids contains three major derivative coumpounds: curcumin (diferuloylmethane, curcumin I, CAS number: 458-37-7), demethoxycurcumin (4-hydroxycinnamoyl(feroyl)methane, curcumin II, CAS number: 24939-17-1), and bisdemethoxycurcumin (Bis(4-hydroxycinnamoyl)methane, curcumin III, CAS number: 24939-16-0). Those compounds have the formula as follows: 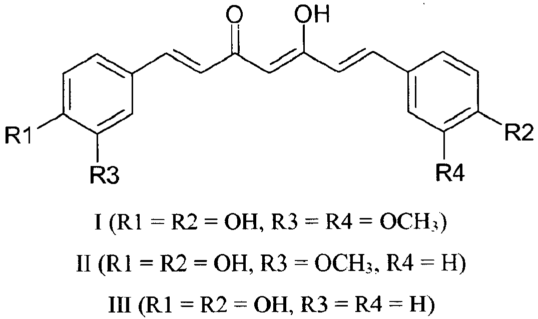 Curcuminoids can exist in at least two tautomeric forms, keto and enol. They may differ from curcumin by the number of ethylene units (one or two), the number of conjugated aromatic rings (one or two), the number of phenol moeties on each ring (one or two), the number and nature of additional substituents (usually alkyloxy derivatives, methoxy in curcumin) or by a combination of the foregoing.

As used herein, the term "cancer" includes any type of cancers or tumors and notably includes solid tumours and leukemias. Examples of cancer that are contemplated by the invention include but are not limited to breast, prostate, ovarian, brain or colon cancers. Examples of tumour metastases that are contemplated by the invention include but are not limited to breast and prostate metastases. Preferably, the cancer and tumour metastases that are contemplated by the invention are advanced primary tumours and metastases.

In a preferred embodiment, cancer or tumour metastases that are contemplated by the invention are Pgp-resistant cancers or tumour metastases. Indeed, the inventors provide evidence that curcumin may limit or inhibit the Pgp extrusion of DTX out of tumour cells and therefore limit or inhibit the drug resistance of said tumour cells.

In another preferred embodiment, cancer and tumour metastases that are contemplated by the invention are primary brain tumours. Indeed, the inventors provide evidence that curcumin may limit or inhibit Pgp transport function in tumor and non-tumor cells and accordingly, the penetration of Pgp-effluxed DTX in Pgp-overexpressing tumor and across endothelial cells of the blood-brain barrier. Accordingly the invention offers to the clinician the feasibility to use DTX for the treatment of brain cancer and brain metastases. Example of brain cancer includes but is not limited to glioblastoma.

Another object of the invention relates to the treatment of a cancer or tumour metastases in a patient in need thereof comprising the step of administering to said patient a therapeutically effective amount of a curcuminoid as defined above and DTX.

According to the invention, the term "patient" or "patient in need thereof" is intended for a human or a non-human mammal.

Generally speaking, a "therapeutically effective amount", or "effective amount", or "therapeutically effective", as used herein, refers to that amount which provides a therapeutic effect for a given condition and administration regimen. This is a predetermined quantity of active material calculated to produce a desired therapeutic effect in association with the required additive and diluent; i.e., a carrier, or administration vehicle. Further, it is intended to mean an amount sufficient to reduce and most preferably prevent a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluents; i.e., carrier, or additive.

In a particular embodiment, curcuminoids of the invention is administered to the patient through any route but preferably orally. Suitable unit oral-route forms include but are not limited to tablets, capsules, powders, granules, oral suspensions or solutions, sublingual and buccal administration forms. Preferably, the curcuminoid compound of the invention is administered through oral capsules, one, two or three-times a day. Preferably, the daily dose of the curcuminoid composition is 6g

DTX is administered to patient through the mode of administration authorized by the regulatory agencies. In a particular embodiment, DTX is administered intravenously at concentration accepted by EMEA for treatment of patients with metastatic breast carcinoma [100 mg/m2] every 21 days, 6 cycles.

In a particular embodiment, curcuminoids of the invention is administered before, concomitantly and after the administration of DTX. In a preferred embodiment, the curcuminoid composition is administered daily for a period of several days during which a single dose of the DTX is administered. According to this embodiment, the curcuminoid compound is administered for 7 days. In a particular embodiment, this cycle of administration (i.e. a period of several days during which the curcuminoid compound is administered daily and a single dose of the DTX is administered) may be repeated. The cycles of administration may be separated by a period during no therapeutic agent is administered. In a particular embodiment the cycle may be repeated 6 times every 21 days.

In a particular embodiment, the active ingredients of the invention are used in combination with one or more pharmaceutically acceptable excipient or carrier. By "physiologically acceptable excipient or carrier" is meant solid or liquid filler, diluents or substances that may be safely used in oral or systemic administration. Depending on the particular route of administration, a variety of pharmaceutically acceptable carriers well known in the art include lipid oil or lipid oil derivatives (cremophor), combination with other lipids, solid or liquid fillers, diluents, hydrotropes, surface active agents, and/or encapsulating substances. Pharmaceutically acceptable carriers for systemic administration that may be incorporated in the composition of the invention include sugar, starches, cellulose, vegetable oils or natural lipids including cremophor or soya phospholipids, buffers, polyols, alginic acid. Representative carriers include membrane lipids, acacia, agar, alginates, hydroxyalkylcellulose, hydroxypropyl methylcellulose, carboxymethylcellulose, carboxymethylcellulose sodium, carrageenan, powdered cellulose, guar gum, cholesterol, gelatin, gum agar, gum arabic, gum karaya, gum ghatti, locust bean gum, octoxynol 9, oleyl alcohol, pectin, poly(acrylic acid) and its homologs, polyethylene glycol, polyvinyl alcohol, polyacrylamide, sodium lauryl sulfate, poly(ethylene oxide), polyvinylpyrrolidone, glycol monostearate, propylene glycol monostearate, xanthan gum, tragacanth, sorbitan esters, stearyl alcohol, starch and its modifications. Suitable ranges vary from about 0.5% to about 1%. For formulating the active ingredients according to the invention, the one skilled in the art will advantageously refer to the last edition of the European pharmacopoeia or the United States pharmacopoeia. Preferably, the one skilled in the art will refer to the fifth edition "2005" of the European Pharmacopoeia, or also to the edition USP 28-NF23 of the United States Pharmacopoeia.

A further object of the invention related to the treatment of breast cancer or breast metastases in a patient in need thereof wherein a cycle consisting of:
- administering orally said patient with curcuminoids of the invention for 7 days
- administering intraveneously said patient with a single dose of DTX 4 days after curcuminoids administration
is repeated 6 times every 21 days.

The invention will be further illustrated by the following figures and examples. However, these examples and figures should not be interpreted in any way as limiting the scope of the present invention.

### EXAMPLE 1: PRECLINICAL STUDIES

### Materials & methods:

**Materials:** Cell culture media were obtained from Gaithersburg, Md. or Life Technologies (Burlington, Ontario, Canada). Fetal bovine calf serum was purchased from Hyclone Laboratories (Logan, UT). Curcumin and DTX were from Sigma (St-Louis, MO). Goat anti-human survivin was from Santa Cruz Biotechnology Inc. (Santa Cruz, CA) and mouse monoclonal antisera directed against HIF-1α from BD Transduction Laboratories (Alphen and Rijn, The Netherlands). Horseradish peroxidase-coupled antimouse secondary antibodies were purchased from Jackson Immuno-Research Laboratories (West Grove, PA) or Dako (Dako, Glostrup, Denmark). Mouse monoclonal anti-β-Actin and cyclosporin A was from Sigma-Aldrich Chimie and enhanced chemoluminescence (ECL) reagents were from Perkin Elmer Life Sciences (Boston, MA). ³[H]-VBL (11.3 Ci/mmol) was from Amersham Pharmacia Biotech (Oakville, Ontario). The intra- and extra-cellular platelet activating factor receptor (PAFR) antagonist: CV-3988 was from Biomol Research Laboratories (Plymouth Meeting, PA).

**Cell culture:** Madine Darby canine kidney (MDCK-MDR1) cells, provided by Amanda Yancy (AstraZeneca Pharmaceuticals), were cultured in DMEM supplemented with 10% FBS as reported previously (Barthomeuf et al. 2005). Human primary fibroblasts were purchased from Biopredic International (Rennes, France). Human MCF7 (ER+ breast), A549 (non small cell lung), PC3 (androgen-resistant prostate) DLD1 (colon), PA1 (ovary), MDA-mb-231 (ER-, PTEN positive), MDA-mb-468 (ER-, PTEN negative) carcinoma and U87 (glioblastoma) lines were purchased from the European Collection of Cell Cultures (ECACC , Salibury, UK) or the American Type Culture Collection (ATCC, Manassas, VA). Cells were cultured in modified Eagle's medium (MEM) supplemented with 10% fetal calf serum, 2 mM L-glutamine and 100 units/ml penicillin, and 100 µg/ml streptomycin (Barthomeuf et al. 2002) or MEM supplemented with 1 mM sodium pyruvate and containing 10% FBS and antibiotics (U-87). The cells were cultured at 37°C under a humidified 95%-5% (v/v) mixture of air and CO₂.

Cell proliferation, attachment and survival: Cells (5,000 cells/well in 96-well plates) were cultured overnight in complete MEM or DMEM medium supplemented with 10% FBS before treatment by curcumin at specified concentration or DTX (1 nM, 5 uM) or curcumin/DTX combination for specified times (6h, 48h or 72h). The drugs were administered in DMSO 0.5 %. Cell proliferation and attachment to the matrix were determined by measuring the amount of resazurin reduced by esterases from living cells (RTT assay) and by measuring the amount of attached biomass with the DNA binding agent (Ho 33342). Fluorescence was assayed at 590/630 nm (RTT assay) or 360/460 nm (Ho assay) as described in (Barthomeuf et al. Planta Med 2002).

Inhibition of [³H]-VBL efflux by MDCKL-MDR1 cells: [³H]-VBL was determined by scintillation counting accordingly to (Barthomeuf et al. Cancer Chemother Pharmacol 2005; 56: 173-81). In brief, confluent MDCK-MDR1 cells were pre-exposed for 30 min to the vehicle (DMSO 0.1%), to curcumin, to cyclosporin A (CsA 10 uM positive control) or to an intra- and extra-cellular platelet activating receptor (PAFR) antagonist CV-3988 (10 uM). The cells were then exposed for 120 min to 20 nM of ³H-VBL (0.23 µCi). ³H-VBL accumulation was stopped by washing the cells (5-fold) with cold PBS (150 mM NaCl, 2.7 mM KCl, 1.3 mM KH₂PO₄, 8.1 mM, Na₂HPO₄, pH 7.4). The cells were lysed with 0.1% Triton X-100 and the radioactivity was counted.

**PAF synthesis:** PAF synthesis was measured by incorporation of [³H]-acetate into lyso-PAF accordingly to the protocol dscribed in (Bernatchez PN et al. Br. J. Pharmacol. 2001 134: 1252 - 1262 and Barthomeuf C. et al. Free Rad Biol Med 2006, 40 : 581 - 590). Briefly, confluent MDCK-MDR1 were exposed to the vehicle or to curcumin at specified concentration for 30 min then rinsed with Hank's balanced salt solution (HBSS) + Hepes (10 mM, pH 7.4) and stimulated for 10 min with 1 ml of HBSS - Hepes buffer (10² M, pH 7. 4) containing CaCl 2 (10² M), [³H]-acetate (2.5 x10⁵ Ci) and PBS. The reaction was stopped by addition of acidified methanol. Polar lipids were isolated according to Bligh and Dyer. After solvent evaporation under N2, ³H-PAF was purified by HPLC on silica gel, and quantified by radioactivity counting vs authentic ³H-PAF.

**Western blot:** After treatment, control and treated cells were washed with PBS, then incubated in PBS containing 1 mM of each NaF and Na₃VO₄ and solubilized on ice in lysis buffer [150 mM NaCl, 10 mM Tris-HCl, pH 7.4, 1 mM EDTA, 1 mM EGTA, 1mM NaF, 1 mM Na₃VO₄, 0.5% (v/v) Nonidet P-40, and 1% (v/v) Triton X-100] or in FosRipa buffer (10 mM Tris/HCl pH 7.5, 250 mM NaCl, 0.1% SDS, 1% Nonidet P40, 1% sodium deoxycholate). When needed insoluble pellets were solubilized in Laemmli samples buffer (62.5 mM Tris-HCl pH 6.8, 2.4% SDS, 100 mM DDT, 10% glycerol, 1 mM EDTA, 0.001% bromphenol blue), heated for 5 min at 96 °C and centrifuged briefly before loading. Protein concentrations and Western blotting was performed according to standard procedures [21,26]. Equal amounts of protein (50 µg) were loaded for separation via 7.5% SDS-PAGE. Mouse monoclonal antisera directed against HIF-1α or survivin were used as primary antibodies and horseradish peroxidase-coupled anti-mouse (Dako) as secondary antibodies.

For determination of HIF-1α levels following curcumin post-treatment, MCF7 cells were treated with DTX at clinical dose, alone or in addition with of the proteasome inhibitor MG132 (10 uM) or with curcumin + MG132 for 6h. Then HIF-1α was assessed by Western blotting.

### Results:

Curcumin inhibits the proliferation of tumor cells exhibiting events correlated with enhanced HIF1 transcription (Pgp or SphK1 overexpression, PTEN inactivation, p53 loss of function): As a first approach to evaluate the interest of curcumin pre- or post- treatment for cancer therapy, we first examined the antiproliferative effect of curcumin on a panel of cancer lines issued from human solid tumors commonly observed in Western countries and characterized by events associated with up-regulation of Akt (rapid proliferation) and of HIF-1 transcription (PTEN inactivation, Pgp or SphK1 over-expression, p53 loss of function).

The human tested lines include highly invasive U87 glioblastoma cells (SphK1 overexpression), 5 human carcinoma lines: weakly invasive MCF7 (estrogen receptor (ER) and PTEN positive) and metastatic MDA-mb-468 (ER and PTEN lack of function) breast carcinoma cells, metastatic PC3 (androgen resistant, p53 loss of function) prostate carcinoma cells, highly proliferative PA1 ovarian carcinoma cells and DLD1 (Pgp-positive) colon carcinoma cells. Primary fibroblasts were used as a model of human non-tumoral cells. Assays were conducted on exponentially growing human cells cultured overnight in MEM supplemented with 10% FBS. The metabolic activity of control and treated cultures was determined with the resazurin reduction test

The profile of toxicity show that at 10 uM, curcumin strongly impairs the growth of all tested tumor lines without significant toxicity for primary fibroblasts. The proliferation of PA1 cells is suppressed and that of other tumor lines decreased from 23% (MDA-mb-468) to 84% (DLD1). This indicates a relative selectivity for tumor cells and show that at tested concentration, curcumin largely overcomes Pgp transport and maintains significant activity on cells expressing hallmarks of resistance to DTX (PTEN inactivation, Pgp expression). This supports a value of curcumin/DTX combination for treatment of tumors displaying these characteristics.

Co-treatment with curcumin significantly enhances DTX efficacy in highly invasive U87 glioblastoma, in metastatic MDA-mb-468 breast carcinoma and in Pgp-positive-DLD1 colon carcinoma cells: Co-treatment by curcumin (10 uM) strongly enhances the toxicity of DTX at low concentration (1 nM) on U87-MG (glioblastoma), MDA-mb-468 (metastatic breast carcinoma) and DLD1 (colon carcinoma) cells. Data show that a 48h co-treatment enhances in a synergistic manner DTX efficacy on U87 and MDA-mb-468 cells and potentiates DTX efficacy in DLD1 colon carcinoma.

Pre-treatment by curcumin enhances (³H)-vinblastine uptake in MDCK-MDR1 cells (a non-tumour line identified as a powerful model for primary evaluation of drug efflux across the blood brain barrier. The reversal effect is dose-dependent and involves inhibition of PAF synthesis: It is established that DTX is largely ineffective on brain tumors (Investigator Brochure, 2002) primarily because DTX cannot cross the blood brain barrier (BBB). At doses susceptible to be observed in man, curcumin pre- and co-treatment dose-dependently accumulates Pgp-transported drugs in MDCK-MDR1 cells, a non-tumor line stably transfected with the human MDR1. Pgp transport requires P-glycoprotein/caveolin-1 interaction (Barakat S et al. Biochem Biophys Res Commun. 2008, 372(3): 440-6). MDCK-MDR1 cells contain functional caveolin and express P-glycoprotein (Pgp) at levels slightly higher than endothelial cells of the BBB (Barakat et al. 2008) and, therefore represent a good model for primary investigation of passage accross the BBB.

In man, the blood levels of curcumin peak 2h after oral administration. After ingestion of 8,000 mg/d, they have been estimated as 1.77 uM (Cheng et al. 2001). Liu et al. have demonstrated that in rats feeding curcumin-phospholipid complex instead of curcumin alone, the blood levels of curcumin are increased about 3-fold (Liu A et al. J Pharmaceut. Biomed. Analysis 2006, 40: 720-27). This suggests that it would be possible to reach blood levels ranging between 1.5 and 5 uM in man. Accordingly, tests were carried out with curcumin at 1.5, 3, 5 and 10 uM.

As taxanes, vinca-alkaloids are rapidly effluxed by Pgp. We have measured over 120 min the accumulation of (³H)-vinblastine ((³H)-VBL) in confluent MDCK-MDR1 cells submitted to a 30-min pre-exposure to the vehicle (DMSO 0.1%), curcumin (0-10 uM) or the reference products: cyclosporin A (CsA, 10 uM) or cnidiadin (Cni, 100 uM) before (³H)-VBL) labeling. We have observed that pre-exposure to 3-10 uM curcumin or to the PAFR inhibitor (CV-3988) promotes (³H)-VBL accumulation in MDCK-MDR1 cells. At 10 uM, curcumin is as efficient as CsA. That uptakes reaching 64% and 36 % of the CsA value are observed after treatment with 5 uM and 3 uM of curcumin, respectively suggests that pre- and post-treatment by curcumin at doses at which curcumin may be observed in man, may efficiently increase DTX penetration into the brain.

The down-regulation of drug efflux is correlated with a down-regulation of PAF-synthesis. As other Pgp-overexpressing cells, ECs of the BBB express Pgp together with another marker of cell resistance: sphingosine kinase-1 (SphK1) (Barthomeuf et al. 2008, Pilorget et al. J Neurochem. 2007 100(5):1203-10). The SphK1 product: sphingosine-1-phosphate (S1P) enhances drug efflux in ECs of the BBB (Pilorget et al. J Neurochem. 2007 100(5):1203-10) and through binding to G-protein dependent receptors found in endothelial and tumor cells enhances angiogenesis and tumor invasiveness and metastasis in part through up-regulation of PAF-dependent events. Our data show that at doses at which it can be observed in man, pre- and co-treatment by curcumin may decrease S1P-mediated events in Pgp-overexpressing cells through down-regulation of PAF synthesis and, accordingly may decrease angiogenesis and tumor proliferation. Since PAF contributes to release of IL-8 involved in conservation of angiogenesis in HIF-1 knowdown tumor cells, curcumin may help decrease both HIF-1-dependent and -independent angiogenesis and Pgp-transport.

Treatment by DTX at clinical dose contributes to induction of drug resistance. This effect can be delayed by curcumin post-treatment. We finally examined the effect of DTX on MCF7 cell survival and HIF-1 transcription. In this aim, DTX was tested at 2 concentrations 1 nM and 5 uM (a concentration representative of blood levels after DTX administration, Investigator Brochure 2002). A1 tests were carried out on highly proliferative cells cultured in MEM medium supplemented with 10% FBS. Survival was assayed with the Ho assay after 6h and 72h.

A 72h-treatment by DTX at clinical dose (5 uM) failed to abolish MCF7 survival but decreased MCF7 survival by 92% (P < 0.01).

The cells surviving treatment by DTX at clinical doses expresses higher amount of survivin than control cells. Survivin is involved in Akt-mediated resistance to DTX (Pen et al. 2006) and is expressed in breast cancer cells at levels correlating with sensitivity to apoptosis (Tanaka K. et al. Clin.Cancer Res. 2000, 6 : 127-34). Our data thus mean that DTX contributes to acquisition of drug resistance by selecting sub-populations expressing higher levels of survivin and maintaining HIF-1 transcription even under normoxia.

Survivin gene expression is transcriptionnally regulated by HIF-1 through PI3K/Akt dependent up-regulation of HIF-1α expression and this effect is up-regulated by EGFR signaling (Pen et al. 2006). As expected the resistant sub-populations treated by DTX at clinical doses expressed higher amounts of HIF-1α than control cells. Surprisingly, the resistant sub-populations treated by DTX at low concentration contained less HIF-1α than control cells. This indicates that Akt is up-regulated in a concentration-dependent manner and probably through activation of distinct pathways. These data suggest two ways for delaying acquisition of resistance and at the same time maintaining high antitumor activity. The first one consist to use inhibitors of Akt signaling (such as curcumin) after treatment with DTX at clinical dose. The second to use DTX at lower concentration in combination with Akt inhibitors and if needed post-treatment by Akt inhibitors.

We verified the value of post-treatment by curcumin against EGF-mediated up-regulation of Akt signaling by examining the levels of HIF-1α synthesis.

In normoxic cells, HIF-1 transcription is normally rapidly suppressed because HIF-1α synthesis is normally rapidly degraded by the proteosomal system (Semenza et al. 2003). Accordingly, addition of inhibitors of proteosomal degradation such as MG-132 (10 uM) leads to HIF-1α accumulation. The level of accumulation vary with the rate of synthesis. We examined the levels of HIF-1α in MCF7 cells treated for 72h by DTX at clinical dose (5 uM) then stimulated for 6h by 10 ng/mL EGF in FBS (10%) in the presence of MG-132 alone with or without curcumin (10 uM). The lower amount of HIF-1α in curcumin-treated cells supports the hypothesis than curcumin may down-regulate EGF-mediated up-regulation of Akt activation and survivin expression.

### Conclusions:

In vitro assays show that :
(i) curcumin displays significant activity on tumor cells exhibiting events positively correlated with enhanced HIF-1 transcription (Pgp expression, PTEN inactivation, p53 loss of function)
(ii) co-treatment with curcumin enhances DTX activity in 3 human tumor cell lines displaying events positively correlated with resistance to DTX: U87 glioblastoma cells (high Sphingosine kinase-1 expression), metastatic MDA-mb-468 breast carcinoma cells (lack of function of PTEN tumor suppressor) and DLD-1 colon carcinoma cells (Pgp expression).
(iii) It is established that DTX is largely ineffective on brain tumors (Investigator Brochure, 2002) primarily because DTX cannot cross the blood brain barrier (BBB). At doses susceptible to be observed in man, curcumin pre- and co-treatment dose-dependently accumulates Pgp-transported drugs in MDCK-MDR1 cells, a non-tumor line stably transfected with the human MDR1 gene. Pgp transport is dependent to P-glycoprotein/caveolin-1 interaction (Barakat S et al. Biochem Biophys Res Commun. 2008, 372(3): 440-6). As MDCK-MDR1 cells contains functional caveolin and express P-glycoprotein (Pgp) at levels slightly higher than endothelial cells of the BBB (Barakat et al. 2008), they represent a good model for primary investigation of drug penetration accross the BBB. Our data support the hypothesis that curcumin may facilitate drug penetration across the blood brain barrier (a pre-requisite of brain tumor treatment.). Based on these data, it can be assumed that curcumin pre- and co-treatment may help reverse brain tumor resistance to DTX.
(iv) It is demonstrated for the first time that at doses susceptible to be observed in man, curcumin reduces Pgp-transport through down-regulation of PAF synthesis. Since HIF-1 transcription leads to up-regulation of Akt and MAPK signaling (Clottles 2005), Akt and MAPK signaling are continuously up-regulated in MDCK-MDR1 cells and other Pgp-overexpressing cells including cells of the BBB. Consequently in these cells, autophagy is down-regulated. PAF enhances angiogenesis, tumor proliferation and metastasis through up-regulation of Akt and MAPK signaling (Axelrad TW et al. FASEB Journal, 2004, 18 : 568-570) and contributes to release of IL8 involved in maintenance of angiogenesis in HIF-1-knowdown tumor cells (Mizukami, Y. et al.. Nat Med. 2005 Sep;11(9):992-7). Curcumin may therefore reduce both Pgp-transport and HIF-1-dependent and -independent angiogenesis through down-regulation of PAF synthesis. This supports a critical role of PAF in tumor progression, angigenesis and induction of resistance.
(v) Finally, data in MCF7 cells have demonstrated for the first time that at clinical doses, DTX directly contributes to acquisition of drug resistance through selection of sub-populations expressing higher amounts of survivin than initial populations. Curcumin post-treatment may partially prevent additional epidermal growth factor (EGF)-mediated up-regulation of HIF-1α expression, delaying acquisition of resistance. As HIF-1α up-regulation is not observed when DTX is used at low doses, another strategy would be to use DTX at lower doses in combination with Akt inhibitors and if needed further treatment by Akt inhibitors.

### EXAMPLE 2: CLINICAL STUDY:

### Protocol:

14 patients (age 53-82, median 71) with metastatic breast adenocarcinoma (WHO performance status ≤ 2 prior therapy regimen, adequate organ function) were enrolled between Jan 2007 and Apr 2008. All have signed informed consent. They were treated in first line chemotherapy by docetaxel (Taxotere®, DTX) accordingly to the schedule of administration accepted by EMEA: IV perfusion 100 mg/m2 every 21 days for 6 cycles.

Curcuminoids were administered orally at 500 mg and escalated until Maximum Tolerated Dose (MTD) was reached. The administered dose varied from 500 mg to 8g/day. Each dose was given to 1 patient per level until a DLT occurred. In this case, the level was documented on at least 3 patients. In practice, curcuminoids were given as follows : 500 mg, 1g, 2g, 4g, 6g or 8g during 7 days, then stopped for 14 days. Ingestion started 4 days before DTX administration, was maintained the day of administration and, finished two days later. The dose-escalation procedure was guided by the "Continual Reassessment Method" (CRM) and based on an inter-patient basis.

No intrapatient dose escalation was used. Each patient received the same dose of curcuminoids (500 to 8,000 mg to) at each course 1 to 3-times/day. Curcuminoids were ingested at breakfast time and, when needed fractionated into 3 fractions ingested at breakfast (and when needed at dinner or at lunch and dinner time) according to the following: 1-0-0 (500 mg), 1-0-1 (1g), 2-0-2 (2g), 3-2-3 (4g), 4-4-4 (6g), 6-4-6 (8g).

The Dose Limiting Toxicity (DLT) was defined according to NCI Common Toxicity Criteria (NCI/CTC) version 3.0, as any non-haematological grade 3-4 toxicity (except alopecia), an absolute neutrophil count grade 4 lasting for at least 7 days, febrile neutropenia grade 4 (defined as an absolute neutrophil count < 1.0 g/L associated with fever ≥ 38.5°C) or thrombocytopenia grade 4. After the 3rd course of DTX without DLT, a new patient was entered at the next dose level.

When a DLT was observed, 2 additional patients were entered at the same dose of curcuminoids to document the toxicity at this level and determine MTD. If no DLT was observed in the 2 patients, dose escalation was continued. When one DLT was observed on at least 1 patient, resulting in 2 DLT out of 3 patients, dose escalation was stopped.

However, as at these doses of DTX, febrile neutropenia is normally observed in about 5% of patients, when febrile neutropenia was the only DLT criteria, the patient number at this level was extended to 5. When DLT other than febrile neutropenia was observed in at least 2 out 3 patients, MTD was defined as the immediately dose level.

As DLT was not observed until the 8 grammes level, at the end of dose escalation, 5 new patients were entered at the last dose level. At this dose 1 DLT was observed, and 2 patients refused to go on with curcuminoids due to the high number of daily caps, and the MTD was reached. Then the recommended dose of curcuminoids was defined as the 6 grammes per day dose-level accordingly to the planned schedule.

The initial protocol and each substantial modification were approved by the Ethics Committee and the Competent Authority/Institutional Review Board (AFSSAPS). The study was conducted accordingly to the Good Clinical Practice requirements.

Serum VEGF and compliance were assessed in 14 patients before each DTX infusion. VEGF was assayed with VEGF ELISA HU (KHG0112, Biosource International/Invitrogen, USA).

Patients were evaluated for haematological and non-hematological toxicities accordingly to NCI-CTC criteria version 3.0, before each DTX course and for clinical efficacy (through measurements of clinical targets and CEA and CA15.3 tumor biomarkers (K-CEA-100, K-C15-3-075, technologie TRACE, Brahms, France) before treatment and after 3 and 6 courses.

### Results:

Due to the number of gelules to be ingested, doses of 8g per day or more were considered as unacceptable. Two patients treated by curcuminoids at the last dose level (8g/day) stopped the treatment for this reason.

One treatment was stopped for allergy and general malaise at the beginning of the second infusion of DTX and was consequently, attributed to the drug.

One patient died abruptly during the first intercourse. The reason was unknown. However, as no clinical toxicity has been detected, the death was not attributed to the combination treatment. Among the 10 patients who completed the treatment, 3 were not fully evaluable (bone metastases only) and 1 had no clinical target (metastase surgically removed). Consequently, 6 patients were evaluable for response.

Patients were evaluated for hematological and non-hematological toxicities after each DTX cycle and for clinical efficacy (clinical targets and biomarkers, CEA and CA 153) before treatment, between the 3^{rd} and the 4^{th} course, and after the 6^{th} course.

Hematological and non-hematological toxicities remained limited. The main side effects were grade 3 toxicities (3 patients), neutropenia grade 4 without fever (1 patient lasting 14 days after DTX infusion) and allergy (1 patient). Other toxicities were an eventual increase of the cutaneous- and conjonctivitis-DTX-dependent side effects. Importantly, no febrile aplasia was observed and only one isolated DLT was observed in the 5^{th} dose level of curcuminoids.

The number of objective responses usually observed after DTX treatment is around 50%. Neither progression nor stabilization was observed with the combination treatment. In contrast, objective clinical responses (5 partial, one minor) were observed in the 6 evaluable patients. Hence the response was considered satisfactory.

In conclusion, the combination treatment was well tolerated and the side effects observed were mild, and corresponded to those usually observed with DTX alone, mainly cutaneous effects and conjonctival irritation. The clinical efficacy justifies further investigations in randomized Phase II trials with 6g curcuminoids /day as the recommended dose. The first multicentric phase II in advanced and metastatic breast cancer patients is currently approved.

## Claims

1. A combination of a curcuminoid and Docetaxel for use in the treatment of cancers and metastases.

2. The combination of a curcuminoid and Docetaxel for use according to claim 1 wherein said curcuminoid is selected from the group consisting of curcumin, demethoxycurcumin and bisdemethoxycurcumin.

3. The combination of a curcuminoid and Docetaxel for use according to claim 1 or 2 wherein said cancer and metastases are advanced primary tumours and metastases.

4. The combination of a curcuminoid and Docetaxel for use according to any of claims 1 to 3 wherein cancers or tumour metastases are Pgp-resistant cancers or tumour metastases.

5. The combination of a curcuminoid and Docetaxel for use according to any of claims 1 to 3 wherein said cancers and metastases are selected from the group consisting of breast, prostate, ovarian, brain and colon cancers and mestastases.

6. The combination of a curcuminoid and Docetaxel for use according to any of claims 1 to 3 wherein said cancer and metastases are brain cancers and brain metastases.

7. The combination of a curcuminoid and Docetaxel for use according to any of claims 1 to 6 wherein said curcuminoid is administered at a daily dose of 6g.

8. The combination of a curcuminoid and Docetaxel for use according to claim 7 wherein said curcuminoid is administered for 7 days.

9. The combination of a curcuminoid and Docetaxel for use according to any of claims 1 to 8 wherein Docetaxel is administered intravenously at concentration of 100 mg/m² every 21 days for 6 cycles.

## Patentansprüche

1. Eine Kombination aus einem Curcuminoid und Docetaxel zur Verwendung in der Behandlung von Krebsformen und Metastasen.

2. Die Kombination aus einem Curcuminoid und Docetaxel zur Verwendung gemäß Anspruch 1, wobei das Curcuminoid ausgewählt ist aus der Gruppe bestehend aus Curcumin, Demethoxycurcumin und Bisdemethoxycurcumin.

3. Die Kombination aus einem Curcuminoid und Docetaxel zur Verwendung gemäß Anspruch 1 oder 2, wobei der Krebs und die Metastasen fortgeschrittene primäre Tumoren und Metastasen sind.

4. Die Kombination aus einem Curcuminoid und Docetaxel zur Verwendung gemäß einem der Ansprüche 1 bis 3, wobei der Krebs oder die Tumormetastasen Pgp-resistente Krebs- oder Tumormetastasen sind.

5. Die Kombination aus einem Curcuminoid und Docetaxel zur Verwendung gemäß einem der Ansprüche 1 bis 3, wobei die Krebsarten und die Metastasen ausgewählt sind aus der Gruppe bestehend aus Brust-, Prostata-, Eierstock-, Gehirn- und Dickdarmkrebsarten und -metastasen.

6. Die Kombination aus einem Curcuminoid und Docetaxel zur Verwendung gemäß einem der Ansprüche 1 bis 3, wobei der Krebs und die Metastasen Gehirnkrebsarten und Gehirnmetastasen sind.

7. Die Kombination aus einem Curcuminoid und Docetaxel zur Verwendung gemäß einem der Ansprüche 1 bis 6, wobei das Curcuminoid in einer Tagesdosis von 6 g verabreicht wird.

8. Die Kombination aus einem Curcuminoid und Docetaxel zur Verwendung gemäß Anspruch 7, wobei das Curcuminoid über 7 Tage verabreicht wird.

9. Die Kombination aus einem Curcuminoid und Docetaxel zur Verwendung gemäß einem der Ansprüche 1 bis 8, wobei Docetaxel intravenös alle 21 Tage über 6 Zyklen in einer Konzentration von 100 mg/m² verabreicht wird.

## Revendications

1. Combinaison d'un curcuminoïde et de docétaxel pour une utilisation dans le traitement des cancers et des métastases.

2. La combinaison d'un curcuminoïde et de docétaxel pour une utilisation selon la revendication 1, dans laquelle ledit curcuminoïde est choisi dans le groupe constitué par la curcumine, déméthoxycurcumine et bisdéméthoxycurcumine.

3. La combinaison d'un curcuminoïde et de docétaxel pour une utilisation selon la revendication 1 ou 2, dans laquelle ledit cancer et les métastases sont des tumeurs primaires avancées et des métastases.

4. La combinaison d'un curcuminoïde et de docétaxel pour une utilisation selon l'une quelconque des revendications 1 à 3, dans laquelle les cancers ou les métastases de tumeurs sont Pgp résistantes.

5. La combinaison d'un curcuminoïde et de docétaxel pour une utilisation selon l'une quelconque des revendications 1 à 3, dans laquelle ledit cancer et les métastases sont sélectionnées dans le groupe constitué par les cancers et métastases du sein, les cancers et métastases de la prostate, les cancers et métastases des ovaires, les cancers et métastases du cerveau et les cancers et métastases du côlon.

6. La combinaison d'un curcuminoïde et de docétaxel pour une utilisation selon l'une quelconque des revendications 1 à 3, dans laquelle ledit cancer et les métastases sont des cancers du cerveau et des métastases cérébrales.

7. La combinaison d'un curcuminoïde et de docétaxel pour une utilisation selon l'une quelconque des revendications 1 à 6, dans laquelle ledit curcuminoïde est administré à une dose journalière de 6g.

8. La combinaison d'un curcuminoïde et de docétaxel pour utilisation selon la revendication 7, dans laquelle ledit curcuminoïde est administrée pendant 7 jours.

9. La combinaison d'un curcuminoïde et docétaxel pour une utilisation selon l'une quelconque des revendications 1 à 8, dans laquelle le docétaxel est administré par voie intraveineuse à une concentration de 100 mg/m² tous les 21 jours pendant 6 cycles.
